(19) Europäisches Patentamt
European Patent Office
Office européen des brevets

(11) EP 0 878 551 B1

(12) EUROPÄISCHE PATENTSCHRIFT

(45) Veröffentlichungstag und Bekanntmachung des Hinweises auf die Patenterteilung:
**12.01.2005 Patentblatt 2005/02**

(51) Int Cl.⁷: $C12Q\ 1/56$, $C07K\ 16/36$

(21) Anmeldenummer: **98107162.4**

(22) Anmeldetag: **20.04.1998**

(54) **Erhöhung der FVII Empfindlichkeit eines Thromboplastinreagenzes**

Enhancement of the factor-VII sensitivity of a thromboplastin reagent

Augmentation de la sensibilité vis-à-vis du facteur VII d'un réactif thromboplastine

(84) Benannte Vertragsstaaten:
**AT BE CH DE ES FR GB IT LI LU NL SE**

(30) Priorität: **17.05.1997 DE 19720853**

(43) Veröffentlichungstag der Anmeldung:
**18.11.1998 Patentblatt 1998/47**

(73) Patentinhaber: **Dade Behring Marburg GmbH 35001 Marburg (DE)**

(72) Erfinder:
• **Wissel, Thomas, Dr.**
  **35094 Lahntal (DE)**
• **Keuper, Hermann, Dr.**
  **35082 Wetter (DE)**
• **Nettelhoff, Hubert, Dr.**
  **35041 Marburg (DE)**
• **Kandel, Heinz-Georg**
  **35083 Wetter (DE)**
• **Muth, Reiner**
  **35041 Marburg (DE)**
• **Kraus, Michael, Dr.**
  **35041 Marburg (DE)**

(56) Entgegenhaltungen:
WO-A-92/18539          WO-A-95/01571
US-A- 4 784 940

• **B J BIEMOND, M LEVI, H TEN CARTE, H R SOULE, L D MORRIS, D L FOSTER, C A BOGOWITZ, T VAN DER POLL, H R BÜLLER, J WOUTER TEN CATE: "Complete Inhibition of Endotoxin-Induced Coagulation Activation in Chimpanzees with a Monoclonal Fab Fragment against Factor VII/VIIa" THROMBOSIS AND HAEMOSTASIS, Bd. 73, Nr. 2, Februar 1995, Seiten 223-230, XP002078209**

• **J CHABBAT, P PORTE, M TELLIER, M STEINBUCH: "Aprotinin is a Competitive Inhibitor of the Factor VIIa-Tissue Factor Complex" THROMBOSIS RESEARCH, Bd. 71, Nr. 3, 1993, Seiten 205-215, XP002078210**

• **G J BROZE, P W MAJERUS: "Purification and Properties of Human Coagulation Factor VII" JOURNAL OF BIOLOGICAL CHEMISTRY, Bd. 255, Nr. 4, 25. Februar 1980, Seiten 1242-1247, XP002078211**

• **K DALAKER, I HJERMANN, H PRYDZ: "A novel form of factor VII in plasma from men at risk from cardiovascular disease" BRITISH JOURNAL OF HAEMATOLOGY, Bd. 61, Nr. 2, Oktober 1985, Seiten 315-322, XP002078212**

• **P M SANDSET, M L LARSEN, U ABILDGAARD, A K LINDAHL, O R ODEGAARD: "Chromogenic substrate assay of extrinsic pathway inhibitor (EPI): levels in the normal population and relation to cholesterol " BLOOD COAGULATION AND FIBRINOLYSIS, Bd. 2, Nr. 3, Juni 1991, Seiten 425-433, XP002078213**

• **PATENT ABSTRACTS OF JAPAN vol. 095, no. 011, 26. Dezember 1995 & JP 07 209297 A (B M L:KK), 11. August 1995**

• **PATENT ABSTRACTS OF JAPAN vol. 009, no. 093 (P-351), 23. April 1985 & JP 59 218960 A (NARAKEN), 10. Dezember 1984**

**Beschreibung**

Die vorliegende Erfindung betrifft ein Verfahren zur Erhöhung der F VII Empfindlichkeit eines Thromboplastinreagenzes wie in den Ansprüchen 1, 9, 11 und 13 definiert.

Die Thromboplastinzeit nach Quick (PT) wird als Suchtest für einen Mangel an Gerinnungsfaktoren im Blut von Patienten eingesetzt. Ebenso wird die PT zur Überwachung der Therapie mit oralen Antikoagulantien eingesetzt. Die bevorzugte PT für normale Blutspender liegt bei 10-14 Sekunden. Die bevorzugte PT für Plasmen mit Faktor VII-Mangel sollte mehr als 60 Sekunden betragen. Die FVII-Empfindlichkeit wird daher als Verhältnis aus der Gerinnungzeit des FVII Mangelplasmas und der Gerinnungszeit von normalen Blutspendern definiert.

Die Erfindung beschreibt ein Verfahren zur Steigerung der Faktor VII Empfindlichkeit von Thromboplastinreagenzien, indem die PT für Plasmen mit Faktor VII-Mangel verlängert wird, ohne die PT für Normalplasmen wesentlich zu verändern.

Das aktive Thromboplastin löst im Plasma die Gerinnung aus und besteht aus einer Lipid- und einer Proteinkomponente. Das Protein, der Tissuefaktor, ist membrangebunden und wird in vielen verschiedenen Geweben gefunden. Die Bindung zwischen Protein und Lipid beruht auf hydrophoben Wechselwirkungen und ist $Ca^{2+}$ unabhängig. Der Proteinanteil besteht aus einem Glykoprotein mit einem Molekulargewicht von 43-53 kDa. Ein Molekül Tissuefaktor kann ein Molekül FVII oder FVIIa binden. Die Bindung von FVII/FVIIa an Tissuefaktor ist $Ca^{2+}$ abhängig. Der Komplex aus Lipid, Tissuefaktor und FVIIa spaltet FX zu FXa und löst somit über die Aktivierung von Prothrombin letztlich die Gerinnung aus.

Das Einsetzen der Gerinnung in einem Plasma 10 bis 14 sec nach Zugabe eines Thromboplastinreagenzes zeigt ein intaktes Gerinnungssystem an. Eine verlängerte Gerinnungszeit weist auf eine Störung hin. Störungen können durch zu geringe Konzentrationen eines oder mehrerer Gerinnungsfaktoren auftreten. Thromboplastinreagenzien verschiedenen Ursprungs unterscheiden sich häufig in ihrer Empfindlichkeit, den Mangel bestimmter Faktoren anzuzeigen. Dies kann zum Teil an Verschleppungen von geringen Mengen der zu bestimmenden Faktoren ins Reagenz liegen. Gebräuchliche Verfahren zur spezifischen Entfernung von geringen Proteinmengen sind i.A. die Immunadsorption, oder, im Falle der Vitamin K-abhängingen Gerinnungsfaktoren wie FII, VII, IX, und X, die Bariumsulfat-Adsorption (WO 90/05740). Bei Reagenzien humanen Ursprungs ist häufig nur die Empfindlichkeit bezüglich des FVII nicht ausreichend.

Wesentliche Faktoren der Gerinnungskaskade sind Proteasen: Faktor VIIa, IXa, Xa, XIa, XIIa, Plasmakallikrein und Thrombin.

Nachteile der bisher beschriebenen Verfahren bestehen zum einen darin, daß FVII an Tissuefaktor bindet und daher die Ausbeute an Tissuefaktor durch ein solches Adsorptionsverfahren reduziert wird , und zum anderen darin, daß beide Verfahren, insbesondere im Produktionsmaßstab einen erheblichen apparativen und zeitlichen Aufwand beanspruchen.

In der vorliegenden Erfindung wird gezeigt, daß es überraschenderweise möglich ist, unter definierten Bedingungen die FVIIa Restaktivität des Reagenzes selektiv zu inhibieren und somit auf einfache Weise zu dem erwünschten Ergebnis zu gelangen, ohne den Tissuefaktor oder andere Faktoren der Gerinnunsgkaskade zu beeinträchtigen. Das als FVII Empfindlichkeit bezeichnete Verhältnis der Gerinnungszeit des FVII Mangelplasmas und der Gerinnungszeit eines Normalplasmas ist bevorzugterweise > 4, besonders bevorzugterweise > 6 und ganz besonders bevorzugterweise > 10.

Der Faktor VIIa besitzt eine Protease-Aktivität und gehört zu der Klasse der Serinproteasen, wie die übrigen Gerinnungsfaktoren IXa, Xa, XIa, XIIa, Plasma Kallikrein und Thrombin. Diesen Gerinnungsfaktoren ist weiterhin gemeinsam, daß sie zunächst als zymogene Form vorliegen und zur Entfaltung der Protease-Aktivität Proteinbindungen gespalten werden müssen.

Es sind eine Vielzahl von Proteaseinhibitoren beschrieben worden, die zumindest die aktiven Formen dieser Zymogene, wie z.B. Thrombin, inhibieren (W.B. Lawson et al. 1982, Folia Haematol. Leipzig 109 (1982) 1, S.52-60). Zu ihnen gehören Sulfonylfluoride wie Phenlymethylsulfonylfluorid (PMSF), p-Aminoethylbenzenesulfonylfluorid (AEBSF), 4-aminophenylmethansulfonylfluorid (p-APMSF), Organofluorophosphate wie Diisopropylfluorophosphat (DFP), Chloromethylketone wie auch Peptide wie Leupeptin oder Proteine der Serpin-Familie wie C1-Inhibitor, Antithrombin III und Aprotinin.

Die Proteaseinhibitoren haben den Nachteil, daß sie nicht nur den verschleppten Faktor FVII bzw FVIIa im PT inhibieren, sondern auch die Gerinnungsfaktoren IXa, Xa, XIa, XIIa, Plasma Kallikrein und Thrombin in der zu bestimmenden Probe. Damit würden sie die Gerinnungszeit verlängern und einen pathologischen Wert vortäuschen. Es ist also zu erwarten, daß diese Inhibitoren nicht geeignet sind, in einem PT-Reagenz eingesetzt zu werden. Überraschenderweise können diese Inhibitoren jedoch unter bestimmten Bedingunen eingesetzt werden, ohne daß die Gerinnungszeiten der Probe verlängert werden, die FVII-Empfindlichkeit des Reagenzes jedoch trotzdem verbessert wird.

Ein weitere Möglichkeit die enzymatisch Aktivität eines Proteins zu inhibieren, besteht in der Herstellung von spezifischen Antikörper, die das aktive Zentum des Proteins inhibieren. Es sind verschiede Antikörper gegen den Gerinnungsfaktor VII erhältlich, von denen mindestens ein polyklonaler Antikörper die enzymatische Aktivität des FVII/FVIIa inhibiert. Die einfache Zugabe des Antikörpers zu einem PT Reagenz sollte nachteilig sein, da der Antikörper auch den FVII/FVIIa der Probe inhibiert. In der vorliegen Erfindung wird gezeigt, daß überraschenderweise ein Konzentrationsbereich für die Zugabe des Anti-FVII-Antikörpers existiert, indem die Gerinnungzeit eines Normalplasmas nicht verlängert wird, aber die Gerinnungzeit eines FVII-Mangelplasmas jedoch deutlich, und somit wird die FVII-Empfindlichkeit des Reagenzes deutlich gesteigert

Es ist auch schon beschrieben, daß Serin-Proteasen oxidativ inhibiert werden können (S.E. Lind et al. 1993, Blood 82, 5 15522-1531). Dieses Verfahren hat neben dem Nachteil, daß die Serin-Proteasen der Gerinnungskaskade in der Probe inhibiert werden, den Nachteil, daß zusätzlich die Fettsäuren der Phospholipide oxidiert werden können (Dasgupta, A. et al. Live Science 1992, 50, 875-882). Die dabei entstehenden Oxidationsprodukte inhibieren die PT ebenfalls (T.W. Barrowcliffe et al. 1975, Thrombos. Diathes. haemorrh. 33, 271).

Überraschenderweise können die Oxidationsprozesse jedoch so gesteuert werden, daß die unerwünschten Nebenreaktionen vernachlässigt werden können, d.h. die Gerinnungeszeit von Normalplasmen nicht verlängert wird, die FVII-Empfindlichkeit des Reagenzes jedoch verbessert wird. Als Oxidationsmittel geeignet sind Hypochlorit, Wasserstoffperoxid, Permanganat, Mangandioxid. Viele Antioxidantien wie z. B. Ascorbinsäure, Thiole, wie z. B. Gluthation, Acetylcystein, Dithiothreitol und Tocopherole und Tocopherolderivate, wie z. B. Di-tert-butyl-p-hydroxyanisol (BHA), Di-tert-butyl-p-kresol (BHT), Trolox und Propylgallat sind in der Lage, in Gegenwart von Metallionen oxidierend zu wirken. Zu den Metallionen, die die Oxidation katalysieren gehören insbesondere Eisen, Kupfer und Zink. Die Metall katalysierte Oxidation ist besondere geeignet, FVII/FVIIa-Restaktivitäten zu inhibieren.

Das Verfahren der vorliegenden Erfindung nutzt einfache Verfahren zur Wärmebehandlung des Gewebeextraktes selektiv, ohne uns damit einen bestimmten Reaktionsmechanismus einzuschränken, könnte die Wirksamkeit des Verfahrens dadurch erklärt werden, daß durch die spezifische Wärmebehandlung Restaktivitäten des FVII/FVIIa beseitigt werden. Die Wärmebehandlung wird vorteilhafterweise für kurze Zeit bei hohen Temperaturen durchgeführt .

Bei niedrigen Temperaturen zwischen +40 und +60 °C ist die Inaktivierung der FVII-Aktivitäten von einer Reihe von zum Teil nicht kontrollierbaren Faktoren, u.a. von den Dimensionen und der Wärmeleitfähigkeit des eingesetzten Gefäßes und der eingesetzten Lösung abhängig. Die Erhitzungszeit kann ebenfalls stark variieren. Die Erhitzungstemperatur liegt vorteilhafterweise zwischen +65 °C und +160 °C, bevorzugterweise zwischen +80 °C und 140 °C, besonders bevorzugterweise zwischen +90 und +120 °C.

Damit die zugeführte Flüssigkeit innerhalb einer möglichst kurzen Zeit auf die jeweilige Erhitzungstemperatur aufgeheizt wird und anschließend in vergleichbar kurzer Zeit wieder abgekühlt werden kann, ist die Apparatur, in der erhitzt wird, zweckmäßigerweise mit mindestens je einem Anschluß für Heiz- und Kühlmedium ausgestattet. Die Apparatur besteht vorteilhafterweise aus drei Bereichen oder Strecken: einer Heizstrecke, in der das Reagenz auf die benötigte Temperatur erhitzt wird, einer Haltestrecke, in der das Reagenz für eine bestimmte Zeit auf dieser Temperatur gehalten wird und einer Kühlstrecke, in der das Reagenz wieder auf eine niedrige Temperatur abgekühlt wird.

Die konstruktive Gestaltung der Wärmeüberträger sollte derart sein, daß die Differenz zwischen der Erhitzungstemperatur für die Lösung und der Temperatur des Heizmediums möglichst klein ist, was wegen der daraus resultierenden geringen Übertemperatur der Produkt berührenden Wandung die größtmögliche Produktschonung zur Folge hat.

Bevorzugt wird ein Verfahren zur kontinuierlichen Kurzzeiterhitzung wie in EP-A 0571771 zur Virusinaktivierung beschrieben.

Die Aufheiz- und/oder Abkühlzeit sollte jeweils weniger als 30 Sekunden, bevorzugt aber weniger als 5 Sekunden betragen. Die Haltezeit kann zwischen 0,1 und 30 Sekunden, bevorzugt aber zwischen 0,5 und 20 Sekunden liegen.

Im folgenden Beispiel wird die Inaktivierung von Restaktivitäten des FVII/FVIIa durch Erhitzung ohne Einschränkung der Erfindung am Beispiel von humanem Plazentaextrakt, wie er für ein gewebethromboplastinhaltiges Quick-Reagenz der Behringwerke AG (Thromborel S, Prod.Nr. OUHP) eingesetzt wird, gezeigt. Vorteilhafterweise wird das Reagenz mit Konservierungsmitteln, Antioxidantien, Kohlenhydraten, Proteinen Aminosäuren und Kombinationen daraus als Stabilisatoren versetzt. Besonders geeignet sind in den einzelnen Substanzklassen folgende Verbindungen:

Konservierungsmittel:
Mergal, Natriumazid Phenol, Amphotericin, Gentamycin, Piperacilin, Ciprofloxacin.

Antioxidantien:
Di-tert-butyl-p-hydroxyanisol (BHA), Di-tert-butyl-p-kresol (BHT), Spermin, Propylgallat, Tocopherole, Trolox, Dithioeritreit, Glutathion.

Kohlenhydrate:
Trehalose, Saccarose, Tylose, Mannit, Gum Xanthan, Phytagel, Caragenan, Polyethylenglykoll.

Proteine:
Lactoglobulin, Lipoproteine und Albumine wie z.B. Serumalbumin, Lactalbumin, Ovalbumin

Aminosäuren:
Acetylcystein, Acetylmethionin, Glycin, Lysin, Histidin, Serin.

Die folgenden Beispiele erläutern die Erfindung.

**Beispiel 1**

Schritt a)

Thromborel S Bulkware wurde mit 1,25ml einer Stabilisierungslösung versetzt und auf 10 ml aufgefüllt. Je 1ml Thromborel (7 °C) wurde in 1,5 ml Reaktionsgefäße gegeben und unterschiedlich lange bei 56 °C in einem Wasserbad inkubiert und anschließend auf Eis abgekühlt.

Schritt b)

Anschließend wurde die Thromboplastinzeit (PT) nach Quick mit einem Schnitger & Gross wie folgt bestimmt:

- Reagenzien u. Röhrchen auf 37°C erwärmen;
- 100µl Kontrollplasma N oder FVII Mangelplasma in die Röhrchen vorlegen;
- 1min im Thermoblock inkubieren;
- 200µl Thromboplastin zugeben und gleichzeitig Meßvorgang starten;
  Röhrchen kurz aufschütteln, in die Meßstelle des Schnitger & Gross stellen und den Elektrodenhalter bis zum Anschlag einsetzen;
- Ablesen der Gerinnungszeit.

Die abgelesenen Gerinnungszeiten sind in Tabelle 1 aufgeführt:

Tab. 1

| PT nach Thermobehandlung | | | | | | |
|---|---|---|---|---|---|---|
| Standardhumanplasma (sek) | | | | F-VII-Mangelplasma (sek) | | |
| min | 1. | 2. | *MW* | | | *MW* |
| Ref. | 10,4 | 10,5 | **10,5** | 28,4 | 27,9 | **28,2** |
| 1 | 10,5 | 10,5 | **10,5** | 28,4 | 27,9 | **28,2** |
| 2 | 10,6 | 10,6 | **10,6** | 29,1 | 29,6 | **29,4** |
| 3 | 10,5 | 10,9 | **10,7** | 33,0 | 33,0 | **33,0** |
| 4 | 10,8 | 10,9 | **10,9** | 34,9 | 34,0 | **34,5** |
| 5 | 11,0 | 11,1 | **11,1** | 36,6 | 36,5 | **36,6** |
| 10 | 12,0 | 11,9 | **12,0** | 45,4 | 44,5 | **45,0** |
| 15 | 14,5 | 15,0 | **14,8** | 116,0 | 116,4 | **116,2** |
| 20 | 15,5 | 16,0 | **15,8** | 128,0 | 126,0 | **127,0** |

**Beispiel 2**

Thromborel S Bulkware wurde mit einer Verweilzeit von 2,05 sec auf Temperaturen von 60 °C bis 80 °C erhitzt (s. Tab. 2).
Die Gerinnungszeit wurde, wie in Beispiel 1 Schritt b) beschrieben, bestimmt.

Tab. 2

| PT nach Kurzzeiterhitzung | | | | | | | | | |
|---|---|---|---|---|---|---|---|---|---|
| | Standardhumanplasma 100% (sek) | | | Standardhumanplasma 25% (sek) | | | F-VII-Mangelplasma 100% (sek) | | |
| | 1. | 2. | *MW* | 1. | 2. | *MW* | 1. | 2. | *MW* |
| Ref | 10,5 | 10,4 | **10,5** | 24 | 24 | **24,0** | 32,6 | 32,5 | **32,6** |
| 60°C | 10,4 | 10,4 | **10,4** | 23,9 | 24,4 | **24,2** | 37,1 | 37 | **37,1** |
| 65°C | 11 | 11 | **11,0** | 25 | 25,8 | **25,4** | 48 | 48,6 | **48,3** |
| 70°C | 11 | 11 | **11,0** | 25,5 | 26 | **25,8** | 59,1 | 59,6 | **59,4** |
| 75°C | 11,4 | 11,4 | **11,4** | 25,9 | 26,5 | **26,2** | 67,6 | 67,1 | **67,4** |
| 80°C | 11,4 | 11,5 | **11,5** | 26,4 | 26,4 | **26,4** | 68,6 | 69,1 | **68,9** |

EP 0 878 551 B1

**Beispiel 3**

Thromborel S Bulkware wurde mit einer Verweilzeit von 0,5 - 2,05 Sek. auf Temperaturen von 75 °C bis 95 °C erhitzt (s. Tab. 3).
Die Gerinnungszeit wurde, wie in Beispiel 1 Schritt b) beschrieben, bestimmt.

Tab. 3

| PT nach Kurzzeiterhitzung | | | | | | |
|---|---|---|---|---|---|---|
| | Standardhumanplasma (sek) | | | F-VII-Mangelplasma (sek) | | |
| | **1.** | **2.** | **MW** | 1. | 2. | **MW** |
| **75°C** | 10,9 | 10,9 | **10,9** | 74,5 | 74,4 | **74,5** |
| **80°C** | 11,1 | 11,1 | **11,1** | 78 | 78,6 | **78,3** |
| **85°C** | 10,9 | 10,9 | **10,9** | 82,9 | 83,9 | **83,4** |
| **90°C** | 10,4 | 10,9 | **10,7** | 82,4 | 82,5 | **82,5** |
| **95°C** | 11 | 11,1 | **11,1** | 79,6 | 79,6 | **79,6** |
| **95°C (1 sek)** | 11 | 10,9 | **11** | 72 | 70,9 | **71,5** |
| **95°C (0,5 sek)** | 11 | 11 | **11** | 67,4 | 68 | **67,7** |
| **Ref** | 10,4 | 10,5 | **10,5** | 35,5 | 35,5 | **35,5** |

**Beispiel 4**

Thromborel S, hergestellt nach Verfahren Beispiel 1 a) - 1 b) wurde mit einer Verweilzeit von 2 - 16 Sek. auf Temperaturen von 65 °C bzw 75 °C erhitzt (s. Tab. 4). Die Gerinnungszeit wurde, wie in Beispiel 1 Schritt b) beschrieben, bestimmt.

Tab. 4

| | Standardhumanplasma 100% (sek) | | | Standardhumanplasma 25% (sek) | | | F-VII-Mangelplasma 100% (sek) | | |
|---|---|---|---|---|---|---|---|---|---|
| **PT nach Kurzzeiterhitzung** | | | | | | | | | |
| | **1.** | **2.** | *MW* | **1.** | **2.** | *MW* | **1.** | **2.** | *MW* |
| **Ausgangsmaterial** | 10,5 | 10,4 | **10,5** | 26,9 | 26,5 | **26,7** | 33,6 | 33,5 | **33,6** |
| **2,0 sek RT** | 10,5 | 10,5 | **10,5** | 26 | 26,4 | **26,2** | 33,1 | 33,5 | **33,3** |
| **15,8 sek 65°C** | 11,4 | 11,5 | **11,5** | 27,5 | 28,5 | **28,0** | 71,6 | 71,5 | **71,6** |
| **7,9 sek 65°C** | 11 | 10,9 | **11,0** | 27,5 | 27,4 | **27,5** | 58,1 | 59,1 | **58,6** |
| **4,0 sek 65°C** | 10,9 | 10,9 | **10,9** | 27,4 | 27 | **27,2** | 50 | 49,6 | **49,8** |
| **3,0 sek 65°C** | 10,9 | 11 | **11,0** | 27 | 26,5 | **26,8** | 48,6 | 48,6 | **48,6** |
| **2,0 sek 65°C** | 10,9 | 11 | **11,0** | 27,5 | 27,9 | **27,7** | 45,1 | 44,6 | **44,9** |
| **3,0 sek 75°C** | 11 | 11,4 | **11,2** | 28,5 | 28,9 | **28,7** | 71,6 | 72,6 | **72,1** |

**Beispiel 5**

Thromborel S, hergestellt nach dem Verfahren in Beispiel 1 a) - 1 b), wurde mit einer Verweilzeit von 3 - 18 Sek. auf eine Temperatur von 90 °C erhitzt (s. Tab. 4). Die Gerinnungszeit wurde, wie in Beispiel 1 Schritt b) beschrieben, bestimmt.

Tab. 5

| PT nach Kurzzeiterhitzung | | | | | |
|---|---|---|---|---|---|
| | Standardhumanplasma (sek) | | | F-VII-Mangelplasma (sek) | |
| | **1.** | **2.** | *MW* | | | *MW* |
| **3 sek** | 13,5 | 13,5 | **13,5** | 101,5 | 102 | **101,8** |
| **6 sek** | 13,5 | 13,4 | **13,5** | 106,5 | 110,5 | **108,5** |
| **9 sek** | 13,6 | 13,6 | **13,6** | 114,6 | 115,1 | **114,9** |
| **12 sek** | 13,5 | 13,5 | **13,5** | 124,9 | 125,4 | **125,2** |
| **15 sek** | 13,4 | 13,9 | **13,7** | 127,4 | 128 | **127,7** |
| **18 sek** | 13,6 | 13,7 | **13,7** | 131,1 | 132 | **131,6** |

**Beispiel 6**

Thromborel S Bulkware wurde mit einer Verweilzeit von 0,5 bis 1,5 Sek. auf eine Temperatur von 120 °C erhitzt. Die Gerinnungszeit wurde mit dem Behring Coagulation Timer mit der Methode PT.sec Thromborel S bestimmt.

Tab. 6

| PT nach Kurzzeiterhitzung bei 120°C | | | |
|---|---|---|---|
| **TB-255** | | | |
| **Verweilzeit in sek:** | **0,5 sek** | **1,0 sek** | **1,5 sek** |
| | **Gerinnungszeit in sek** | | |
| **Standardhumanplasma:** | 11,1 | 11,2 | 11,2 |
| **F-VII-Mangelplasma:** | 120,7 | 146,8 | 155 |

**Beispiel 7**

Thromborel S Bulkware wurde mit einer Verweilzeit von 3 sek auf eine Temperatur von 85 °C erhitzt. Kommerziell erhältliches Thromborel S und erhitztes Thromborel (TB-190) wurden Anhand von verschieden Proben verglichen.
Die Gerinnungszeit wurde, wie in Beispiel 1 Schritt b) beschrieben, bestimmt.

**Proben:**

| | | |
|---|---|---|
| PT100: | Standard-Human-Plasma | Ch.B.:502556 (98% d.N.) |
| Ktrl. P: | Kontrollplasma P | Ch.B.:512628 |
| MACI: | Marcumarplasma I. | Ch.B.:951201 |
| MACII: | Marcumarplasma II. | Ch.B.:951202 |
| PATH-I: | Pathoplasma I. | Ch.B.:502883 |
| PATH-II | Pathoplasma II. | Ch.B.:502973 |
| FVII: | Faktor VII-Mangelplasma | Ch.B.:500756 |

1 IU/ml Hep: Standard-Human-Plasma mit 1 IU/ml Heparin aufgestockt. MAC-02 u. MAC-03: Plasmapools von oral antikoagulierten Patienten 50% FVII, 2% FVII: Verdünnungen von FVII Mangelplasma in Standard-Human-Plasma.

PT16,75: Standard-Humanplasma auf 16,75% in physiologischer NaCl-Lösung verdünnt.

% d. Norm: % der Norm (Abschätzung mit folgendem Zusammenhang:

$$C_x=(PT_{16,75\%}-PT_{100\%})/(4,97*PT_x-4*PT_{100\%}+PT_{16,75\%})$$

ISI: International Sensitivity Index:

PR: $PT_{(Marcumarplasma)}/PT_{(Standard-Human-Plasma)}$

$ISI_k$: ISI des kalibrierten Reagenzes; $ISI_x$: ISI des nicht kalibrierten Reagenzes

$$ISI_k=ISI_x*log(PR_x)/log(PR_k)$$

## Tab. 7

| PT nach Kurzzeiterhitzung | | | | | | | | |
|---|---|---|---|---|---|---|---|---|
| | Thromborel S | | | | TB-190 | | | |
| | | % d. N. | PR | INR | | % d. N. | PR | ISI |
| PT100 | 11,15 | | | | 10,05 | | | |
| PT16,75 | 41,9 | | | | 46,2 | | | |
| 1IU/ml Hep. | 12,25 | 84,9% | 1,10 | 1,11 | 10,1 | 99,3% | 1,00 | |
| MACI | 34,95 | 20,6% | 3,13 | 3,43 | 31,2 | 25,6% | 3,10 | 1,09 |
| MACII | 66 | 10,1% | 5,92 | 6,82 | 67,8 | 11,2% | 6,75 | 1,01 |
| MAC-02 | 42,2 | 16,6% | 3,78 | 4,21 | 39,9 | 19,6% | 3,97 | 1,04 |
| MAC-03 | 60,9 | 11,1% | 5,46 | 6,26 | 58,4 | 13,1% | 5,81 | 1,04 |
| PATH-I | 29,25 | 25,5% | 2,62 | 2,83 | 25,2 | 32,4% | 2,51 | |
| PATH-II | 51,45 | 13,3% | 4,61 | 5,21 | 44,75 | 17,3% | 4,45 | |
| Ktrl. P | 21,2 | 38,1% | 1,90 | 2,00 | 20,8 | 40,4% | 2,07 | |
| 50% FVII | 12,65 | 80,5% | 1,13 | 1,15 | 11,45 | 83,9% | 1,14 | |
| 2% FVII | 31,1 | 23,7% | 2,79 | 3,03 | 39,45 | 19,8% | 3,93 | |
| FVII | 47,75 | 14,5% | 4,28 | 4,81 | 138,5 | 5,4% | 13,78 | |

**Beispiel 8:**

a)

Thromborel S Bulkware wird kurzzeiterhitzt (Temperatur 95 °C, Verweilzeit 0,5 Sek.) und Aliquots wurden mit verschiedenen Proteaseinhibitoren versetzt (Tabelle 8). Nach Inkubation über Nacht wurden die Stabilisatoren hinzugegeben. Die Referenz enthält keinen Inhibitor.

b)

Anschließend wird die Thromboplastinzeit(PT) nach Quick mit einem Schnitger & Gross wie folgt bestimmt:

- Reagenzien u. Röhrchen auf 37 °C erwärmen;
- 100 µl Plasma in die Röhrchen vorlegen;
- 1 min im Thermoblock inkubieren;
- 200 µl Thromboplastin zugeben und gleichzeitig Meßvorgang starten;
  Röhrchen kurz aufschütteln, in die Meßstelle des Schnitger & Gross stellen und den Elektrodenhalter bis zum Anschlag einsetzen;
- Ablesen der Gerinnungszeit.

Die abgelesenen Gerinnungszeiten sind in Tabelle 8 aufgeführt:

Als Proben wurden Standardhumanplasma (Behring Diagnostics) mit einer Gerinnungsaktivität von ca. 100 %, eine 1:4 Verdünnung des Standard-Human-Plasmas (StHPl) in physiologischer Kochsalzlösung (25 %) und ein FVII-Mangelplasma (FVII-MP, Behring Diagnostics) eingesetzt. Aus dem Verhältnis der Gerinnungszeiten für die Verdünnung des StHPls und dem StHPI wurde die Spreizung berechnet und aus dem Verhältnis der Gerinnungszeiten für FVII-MP und STHPL wurde die FVII-Empfindlichkeit berechnet.

Tabelle 8

| Protease-Inhibitoren | Konzentration | 100% | 25% | FVII-MP | Spreizung: 25%/ 100% | FVII-Empf.: FVII-MP/100% |
|---|---|---|---|---|---|---|
| | | Gerinnungszeiten in sek | | | | |
| pa-PMSF | 0,01mM | 10,6 | 22,3 | 144 | **2,1** | **13,6** |
| pa-PMSF | 0,025mM | 10,6 | 21,8 | 147 | **2,1** | **13,8** |
| pa-PMSF | 0,05mM | 10,6 | 22,2 | 161 | **2,1** | **15,2** |
| pa-PMSF | 0,1mM | 10,7 | 21,7 | 172 | **2,0** | **16,1** |
| pa-PMSF | 0,5mM | 10,8 | 23,1 | 298 | **2,1** | **27,6** |
| PMSF | 1,5mM | 10,7 | 21,9 | 259 | **2,0** | **24,2** |
| Leupeptin | 0,5mg/l | 10,6 | 22,5 | 121 | **2,1** | **11,4** |
| Pepstatin | 0,7mg/l | 10,6 | 21,6 | 115 | **2,0** | **10,9** |
| Antagosan | 100KIE/ml | 10,6 | 22,2 | 118 | **2,1** | **11,1** |
| Referenz | | 10,7 | 21,4 | 135 | **2,0** | **12,6** |
| | | | | | | |
| | | | | | | |

**Beispiel 9:**

Thromborel S Bulkware gemäß Beispiel 8 a), kurzzeiterhitzt bei einer Temperatur von 85 °C und einer Verweilzeit von 3 Sek. wurde mit Stabilisatoren versetzt und zu einigen Aliquots wurden zusätzlich verschiedene Konzentrationen des Proteaseinhibitors Diisopropylfluorophosphat (DFP) gegeben; die Referenz enthält keinen Inhibitor.

Die Gerinnungszeit wurde mit dem Behring Coagulation Timer (PT.sec Thromborel S) bestimmt. Als Proben wurden Standardhumanplasma, das Kontrollplasma Patho II (Behringwerke AG) und ein FVII Mangelplasma eingesetzt.

Aus dem Verhältnis der Gerinnungszeiten des Patho II und dem StHPI wurde die Spreizung berechnet und aus dem Verhältnis der Gerinnungszeiten für FVII-MP und STHPL wurde die FVII-Empfindlichkeit berechnet.

Tabelle 9

| Protease-Inhibitoren | Konzentration | 100% | Patho II | FVII-MP | Spreizung: Patho II/100% | FVII-Empf.: FVII-MP/100% |
|---|---|---|---|---|---|---|
| | | Gerinnungszeiten in sek | | | | |
| DFP | 1mM | 10,0 | 41,9 | 389 | 4,2 | 38,9 |
| DFP | 0,33mM | 9,9 | 40,2 | 331 | 4,1 | 33,5 |

Tabelle 9   (fortgesetzt)

| Protease-Inhibitoren | Konzentration | 100% | Patho II | FVII-MP | Spreizung: Patho II/100% | FVII-Empf.: FVII-MP/100% |
|---|---|---|---|---|---|---|
| | | Gerinnungszeiten in sek | | | | |
| DFP | 0,1mM | 9,9 | 40,7 | 213 | 4,1 | 21,6 |

**Beispiel 10:**

Herstellung eines Anti-FVII-Antikörpers

Das FVII Immunisierungsantigen wurde aus humanem Plasma isoliert. Nach Aluminiumhydroxyd-Adsorption wurden die gebundenen Proteine mit 0,3 M Natriumphosphat, pH 7,4 eluiert. Nach einer anschließenden Fällung mit 16%igem Ethanol wurde der Überstand erneut mit 25 %igem Ethanol gefällt und der Niederschlag mit AE-Cellulose weiter aufgereinigt. Die adsorbierten Proteine wurden mit einer Lösung, die 3% Natriumcitrat, 0,15 M Natriumchlorid und 0,09% EDTA pH 8,0 enthielt, eluiert. Nach weiteren Aufreinigungen mit PVC-Elektrophorese und einer Säulenchromatographie mit Sephadex-G 100 wurde der reine FVII zur Immunisierung eingesetzt.

Nach Standardmethoden wurden Schafe immunisiert und der Anti-FVII-Antikörper aus dem Serum gewonnen.

Thromborel S Bulkware gemäß Beispiel 8a) wurde mit einer Verweilzeit von 3 Sek. kurzzeiterhitzt auf eine Temperatur von 85 °C und zusätzlich mit verschiedenen Konzentrationen des oben beschrieben polyklonalen Antikörpers versetzt.

Die Gerinnungszeit wurde mit dem Behring Coagulation Timer (PT.sec Thromborel S) bestimmt. Als Proben wurden Standardhumanplasma, das Kontrollplasma Patho II und ein FVII Mangelplasma eingesetzt. Aus dem Verhältnis der Gerinnungszeiten des Patho II und dem STHPI wurde die Spreizung berechnet und aus dem Verhältnis der Gerinnungszeiten für FVII-MP und STHPL wurde die FVII-Empfindlichkeit berechnet.

Tabelle 10

| Antikörper | Konzentration µg/ml | 100% | Patho II | FVII-MP | Spreizung: Patho II/100% | FVII-Empf.: FVII-MP/100% |
|---|---|---|---|---|---|---|
| | | Gerinnungszeiten in sek | | | | |
| AK | 6,25 | 10,1 | 42,3 | 400 | 4,2 | 39,8 |
| AK | 0,625 | 10,0 | 41,2 | 301 | 4,1 | 30,2 |
| AK | 0,0625 | 9,9 | 40,4 | 163 | 4,1 | 16,5 |
| Referenz | 0 | 9,9 | 39,2 | 136 | 4,0 | 13,8 |

**Beispiel 11:**

Thromborel S Bulkware gemäß Beispiel 8 a) wurde kurzzeiterhitzt mit einer Verweilzeit von 3 Sek. auf eine Temperatur von 85 °C und zusätzlich mit Ascorbinsäure, Ascorbinsäure und Eisen sowie Ascorbinsäure und Kupfer versetzt. Nach einer inkubation von 1 bis 6 Stunden wurden die Stabilisatoren zugegeben.
Die Gerinnungszeit wurde wie in Beispiel 8b) beschrieben, bestimmt.

Als Proben wurden Standardhumanplasma, das Kontrollplasma Patho II und ein FVII Mangelplasma eingesetzt. Die Reagenzien wurden bei 4 °C und 37 °C gelagert und die Gerinnungszeit nach 7 und 14 Tagen wiederhohlt. Aus dem Verhältnis der Gerinnungszeiten des Patho II und dem STHPI wurde die Spreizung berechnet und aus dem Verhältnis der Gerinnungszeiten für FVII-MP und STHPL wurde die FVII-Empfindlichkeit berechnet.

## Tabelle 11

| Probe: | | SHPL | | |
|---|---|---|---|---|
| **Konzentrationen in mM** | | | | |
| Ascorbinsäure: | 0 | 1 | 1 | 1 |
| Fe(II)SO4: | 0 | 0 | 0,01 | 0 |
| CuCl: | 0 | 0 | 0 | 0,01 |
| Stabilisierung: | nach 6h | nach 6h | nach 1h | nach 6h |
| | | | | |
| Probe: | 100% | | | |
| 18h /4°C | 12,9 | 13,7 | 15 | 13,7 |
| 7 Tage: 4°C | 12,9 | 14 | 15,9 | 13,8 |
| 7 Tage: 37°C | 13,4 | 14,7 | 16,4 | 14,6 |
| 14 Tage: 4°C | 12,9 | 14,1 | 16 | 13,9 |
| 14 Tage: 37°C | 14,8 | 15,3 | 17,1 | 15,5 |
| | | | | |
| Probe: | FVII | | | |
| 18h /4°C | 126,4 | 157,7 | 139,8 | 243,3 |
| 7 Tage: 4°C | 118,8 | 154,3 | 153,2 | 240,8 |
| 7 Tage: 37°C | 114,7 | 181,1 | 167,5 | 261,7 |
| 14 Tage: 4°C | 118,2 | 161,6 | 157,9 | 245,8 |
| 14 Tage: 37°C | 120,2 | 195,8 | 179 | 258,1 |
| | | | | |
| Probe: | Patho II | | | |
| 18h /4°C | 47,6 | 51,4 | 62,7 | 48,5 |
| 7 Tage: 4°C | 46,6 | 51,3 | 64,2 | 48,2 |
| 7 Tage: 37°C | 48,6 | 51,5 | 60,3 | 48,8 |
| 14 Tage: 4°C | 46 | 51,1 | 64,2 | 48,1 |
| 14 Tage: 37°C | 57 | 53,1 | 62 | 53,6 |
| | | | | |
| FVII-Empf.: | FVII/100% | | | |
| + 18h /4°C | 9,8 | 11,5 | 9,3 | 17,8 |
| 7 Tage: 4°C | 9,2 | 11,0 | 9,6 | 17,4 |
| 7 Tage: 37°C | 8,6 | 12,3 | 10,2 | 17,9 |
| 14 Tage: 4°C | 9,2 | 11,5 | 9,9 | 17,7 |
| 14 Tage: 37°C | 8,1 | 12,8 | 10,5 | 16,7 |
| | | | | |
| Spreizung: | Patho II/100% | | | |
| 18h /4°C | 3,7 | 3,8 | 4,2 | 3,5 |
| 7 Tage: 4°C | 3,6 | 3,7 | 4,0 | 3,5 |
| 7 Tage: 37°C | 3,6 | 3,5 | 3,7 | 3,3 |

**Beispiel 12:**

In Thromborel S Bulkware gemäß Beispiel 8a), kurzzeiterhitzt mit einer Verweilzeit von 0,75 Sek. auf eine Temperatur von 120 °C und mit 0,16 mM Natriumcarbonat der pH 7,5 eingestellt, anschließend wurden verschiedene Konzentrationen $H_2O_2$ zugegeben und 90 min bei 37 °C inkubiert. Die Lösung wurde mit Stabilisatoren und 6 mM Acetylcystein versetzt. Der pH-Wert wurde auf 6,5 eingestellt und die Gerinnungszeit gemäß Beispiel 8b) bestimmt.
Als Proben wurden Standardhumanplasma, das Kontrollplasma und ein FVII Mangelplasma eingesetzt. Aus dem Verhältnis der Gerinnungszeiten für FVII-MP und STHPL wurde die FVII-Empfindlichkeit berechnet.

Tabelle 12

| Konzentration $H_2O_2$ | Gerinnungszeit in sek | | FVII-Empf.: |
|---|---|---|---|
| | 100% | FVII-MP | FVII-MP/100% |
| 0,3% | 15 | 156 | 10,4 |
| 0,03% | 11,5 | 143 | 12,4 |
| 0,003% | 11,4 | 139 | 12,2 |
| 0,0003% | 11,7 | 133 | 11,4 |
| 0,00003% | 11,9 | 132 | 11,1 |
| ------- | 11,9 | 131 | 11,0 |
| | | | |
| | | | |

**Beispiel 13:**

In Thromborel S Bulkware gemäß Beispiel 8a), kurzzeiterhitzt bei einer Temperatur von 120 °C und bei einer Verweilzeit von 0,75 Sek., wurde mit 0,16 mM Natriumcarbonat der pH 7,5 eingestellt, anschließend wurden verschiedene Konzentrationen Chloramin T zugegeben und 90 min bei 37 °C inkubiert. Die Lösung wurde mit Stabilisatoren und 6 mM Acetylcystein versetzt. Der pH-Wert wurde auf 6,5 eingestellt und die Gerinnungszeit wurde mit dem Behring Coagulation Timer (PT.sec Thromborel S) bestimmt.
Als Proben wurden Standardhumanplasma, das Kontrollplasma Patho II und ein FVII Mangelplasma eingesetzt. Aus dem Verhältnis der Gerinnungszeiten des Patho II und dem StHPI wurde die Spreizung berechnet und aus dem Verhältnis der Gerinnungszeiten für FVII-MP und STHPL wurde die FVII-Empfindlichkeit berechnet.

Tabelle 13

| Konzentration Chloramin T | Gerinnungszeit in sek | | | Spreizung: Patho II/100% | FVII-Empf.: FVII-MP/100% |
|---|---|---|---|---|---|
| | 100% | Patho II | FV-MP | | |
| 0,1mM | 13 | 38,2 | 177,4 | 2,9 | 13,6 |
| 0,01mM | 11,7 | 34,7 | 141,6 | 3,0 | 12,1 |
| 0,001mM | 11,5 | 34,2 | 140,1 | 3,0 | 12,2 |
| ------- | 11,6 | 34,2 | 142 | 2,9 | 12,2 |
| | | | | | |
| | | | | | |

**Patentansprüche**

1. Verfahren zur Erhöhung der FVII Empfindlichkeit eines Thromboplastinreagenzes **dadurch gekennzeichnet, daß** die FVII/FVIIa Restaktivität des Reagenzes selektiv inhibiert wird, indem eine Lösung dieses Reagenzes auf über 65 °C erhitzt wird.

2. Verfahren nach Anspruch 1 **dadurch gekennzeichnet, daß** eine Lösung von Gewebe-Thromboplastin humanen Ursprungs erhitzt wird.

3. Verfahren nach Anspruch 1 **dadurch gekennzeichnet, daß** eine Lösung eines rekombinanten Gewebe-Thromboplastins erhitzt wird

4. Verfahren nach Anspruch 1 **dadurch gekennzeichnet, daß** eine Lösung einer biologisch aktiven Variante eines Gewebe-Thromboplastins eingesetzt wird.

5. Verfahren nach einem der Ansprüche 1 bis 4 **dadurch gekennzeichnet, daß** in einem Wärmeüberträger mit indirekter Beheizung kontinuierlich und kurzzeitig erhitzt wird.

6. Verfahren nach einem der Ansprüche 1 bis 5 **dadurch gekennzeichnet, daß** die Erhitzungstemperatur zwischen +65 °C und +160 °C liegt.

7. Verfahren nach einem der Ansprüche 1 bis 6 **dadurch gekennzeichnet, daß** die Aufheiz- oder Abkühlzeit kleiner 30 Sekunden ist.

8. Verfahren nach Anspruch 1 **dadurch gekennzeichnet, daß** die Haltezeit zwischen 0,1 und 30 Sekunden liegt.

9. Verfahren zur Erhöhung der FVII Empfindlichkeit eines Thromboplastinreagenzes **dadurch gekennzeichnet, daß** die FVII/FVIIa Restaktivität des Reagenzes selektiv inhibiert wird, indem das Reagenz mit Sulfonylfluoriden, Organofluorophosphaten oder Chloromethylketonen als Proteaseinhibitoren versetzt wird.

10. Verfahren nach Anspruch 9 **dadurch gekennzeichnet, daß** als Proteasinhibitoren Phenlymethylsulfonylfluorid (PMSF), p-Aminoethylbenzenesulfonylfluorid (AEBSF), 4-aminophenylmethansulfonylflurid (p-APMSF) oder Diisopropylfluorophosphat (DFP) oder Kombinationen dieser Inhibitoren eingesetzt werden.

11. Verfahrenzur Erhöhung der FVII Empfindlichkeit eines Thromboplastinreagenzes **dadurch gekennzeichnet, daß** die FVII/FVIIa Restaktivität des Reagenzes selektiv inhibiert wird, indem das Reagenz mit Anti-FVII-Antikörpern versetzt wird, die die enzymatische Aktivität von FVII/FVIIa inhibieren.

12. Verfahren nach Anspruch 11 **dadurch gekennzeichnet, daß** als Antikörper ein polyklonaler Anti-FVII-Antikörper eingesetzt wird.

13. Verfahren zur Erhöhung der FVII Empfindlichkeit eines Thromboplastinreagenzes **dadurch gekennzeichnet, daß** die FVII/FVIIa Restaktivität des Reagenzes selektiv inhibiert wird, indem das Reagenz mit Oxidationsmitteln versetzt wird.

14. Verfahren nach Anspruch 13 **dadurch gekennzeichnet, daß** die Oxidation in Gegenwart der Metalle Eisen, Kupfer oder Zink durchgeführt wird.

15. Verfahren nach Anspruch 14 **dadurch gekennzeichnet, daß** zur Oxidation Ascorbinsäure, Thiole, Tocopherole oder Tocopherolderivate verwendet werden.

16. Verfahren nach Anspruch 13 **dadurch gekennzeichnet, daß** als Oxidationsmittel Chloramin T, Wasserstoffperoxid oder ihre Kombinationen eingesetzt werden.

17. Verfahren nach Anspruch 9, 11 oder 13 **dadurch gekennzeichnet, daß** als Thromboplastinreagenz eine Lösung von Gewebe-Thromboplastin humanen Ursprungs eingesetzt wird.

18. Verfahren nach Anspruch 9, 11 oder 13 **dadurch gekennzeichnet, daß** eine Lösung eines rekombinanten Gewebe-Thromboplastins eingesetzt wird.

19. Verfahren nach Anspruch 9, 11 oder 13 **dadurch gekennzeichnet, daß** eine Lösung einer biologisch aktiven Variante eines Gewebe-Thromboplastins eingesetzt wird.

**Claims**

1. A process for increasing the FVII sensitivity of a thromboplastin reagent, which comprises selectively inhibiting the residual FVII/FVIIa activity in the reagent by a solution of this reagent being heated to greater than 65°C.

2. The process as claimed in claim 1, wherein a solution of tissue thromboplastin of human origin is heated.

3. The process as claimed in claim 1, wherein a solution of a recombinant tissue thromboplastin is heated.

4. The process as claimed in claim 1, wherein a solution of a biologically active variant of a tissue thromboplastin is employed.

5. The process as claimed in one of claims 1 to 4, wherein heating is carried out continuously and for a short time in a heat transfer apparatus having indirect heating.

6. The process as claimed in one of claims 1 to 5, wherein the heating temperature is between +65°C and +160°C.

7. The process as claimed in one of claims 1 to 6, wherein the heating or cooling time is less than 30 seconds.

8. The process as claimed in claim 1, wherein the holding time is between 0.1 and 30 seconds.

9. A process for increasing the FVII sensitivity of a thromboplastin reagent, which comprises selectively inhibiting the residual FVII/FVIIa activity in the reagent by the reagent being admixed with sulfonyl fluorides, organofluorophosphates or chloromethyl ketones as protease inhibitors.

10. The process as claimed in claim 9, wherein phenylmethylsulfonyl fluoride (PMSF), p-aminoethylbenzenesulfonyl fluoride (AEBSF), 4-aminophenylmethanesulfonyl fluoride (p-APMSF) or diisopropyl fluorophosphate (DFP), or combinations of these inhibitors, are employed as protease inhibitors.

11. A process for increasing the FVII sensitivity of a thromboplastin reagent, which comprises selectively inhibiting the residual FVII/FVIIa activity in the reagent by the reagent being admixed with anti-FVII-antibodies which inhibit the enzyme activity of FVII/FVIIa.

12. The process as claimed in claim 11, wherein a polyclonal anti-FVII antibody is employed as antibody.

13. A process for increasing the FVII sensitivity of a thromboplastin reagent, which comprises selectively inhibiting the residual FVII/FVIIa activity in the reagent by the reagent being admixed with oxidizing agents.

14. The process as claimed in claim 13, wherein the oxidation is carried out in the presence of the metals iron, copper or zinc.

15. The process as claimed in claim 14, wherein ascorbic acid, thiols, tocopherols or tocopherol derivatives are used for the oxidation.

16. The process as claimed in claim 13, wherein chloramine T, hydrogen peroxide, or their combinations, are employed as oxidizing agents.

17. The process as claimed in claim 9, 11 or 13, wherein a solution of tissue thromboplastin of human origin is employed as the thromboplastin reagent.

18. The process as claimed in claim 9, 11 or 13, wherein a solution of a recombinant tissue thromboplastin is employed.

19. The process as claimed in claim 9, 11 or 13, wherein a solution of a biologically active variant of a tissue thromboplastin is employed.

**Revendications**

1. Procédé pour augmenter la sensibilité d'un réactif thromboplastine vis-à-vis du facteur VII, **caractérisé en ce que** l'activité résiduelle en FVII/FVIIa du réactif est inhibée sélectivement,une solution de ce réactif étant chauffée à plus de 65 °C.

2. Procédé selon la revendication 1, **caractérisé en ce que** l'on chauffe une solution de thromboplastine tissulaire d'origine humaine.

3. Procédé selon la revendication 1, **caractérisé en ce que** l'on chauffe une solution d'une thromboplastine tissulaire recombinante.

4. Procédé selon la revendication 1, **caractérisé en ce que** l'on utilise une solution d'une variante biologiquement active d'une thromboplastine tissulaire.

5. Procédé selon l'une quelconque des revendications 1 à 4, **caractérisé en ce que** l'on effectue le chauffage brièvement et en continu dans une unité de transfert de chaleur à chauffage indirect.

6. Procédé selon l'une quelconque des revendications 1 à 5, **caractérisé en ce que** la température de chauffage est comprise entre +65 °C et +160 °C.

7. Procédé selon l'une quelconque des revendications 1 à 6, **caractérisé en ce que** les temps de montée en température ou de refroidissement sont inférieurs à 30 secondes.

8. Procédé selon la revendication 1, **caractérise en ce que** le temps de tenue est compris entre 0,1 et 30 secondes.

9. Procédé pour augmenter la sensibilité d'un réactif thromboplastine vis-à-vis du facteur VII **caractérisé en ce que** l'activité résiduelle en FVII/FVIIa du réactif est inhibée sélectivement, le réactif étant additionné de fluorures de sulfonyle, de fluorophosphates organiques ou de chlorométhylcétones à titre d'inhibiteurs de protéase.

10. Procédé selon la revendication 9, **caractérisé en ce que**, comme inhibiteur de protéase, on utilise du fluorure de phénylméthylsulfonyle (PMSF), du fluorure de p-aminoéthylbenzènesulfonyle (AEBSF), du fluorure de 4-aminophénylméthanesulfonyle (p-APMSF), ou du fluorophosphate de diisopropyle (DFP), ou des combinaisons de ces inhibiteurs.

11. Procédé pour augmenter la sensibilité d'un réactif thromboplastine vis-à-vis du facteur VII **caractérisé en ce que** l'activité résiduelle en FVII/FVIIa du réactif est inhibée sélectivement, le réactif étant additionné d'anticorps anti-FVII, qui inhibent l'activité enzymatique FVII/FVIIa.

12. Procédé selon la revendication 11, **caractérisé en ce qu'**un anticorps polyclonal anti-FVII est utilisé comme anticorps.

13. Procédé pour augmenter la sensibilité d'un réactif thromboplastine vis-à-vis du facteur VII, **caractérisé en ce que** l'activité résiduelle en FVII/FVIIa du réactif est inhibée sélectivement, le réactif étant additionné d'agents oxydants.

14. Procédé selon la revendication 13, **caractérisé en ce que** l'oxydation est effectuée en présence des métaux fer, cuivre ou zinc.

15. Procédé selon la revendication 14, **caractérisé en ce que**, pour l'oxydation, sont utilisés l'acide ascorbique, des thiols, des tocophérols ou des dérivés de tocophérols.

16. Procédé selon la revendication 13, **caractérisé en ce que**, pour l'oxydation, sont utilisées la chloramine T, l'eau oxygénée ou leurs combinaisons.

17. Procédé selon l'une quelconque des revendications 9, 11 ou 13, **caractérisé en ce que** comme réactif thromboplastine, on utilise une solution de thromboplastine tissulaire d'origine humaine.

18. Procédé selon l'une quelconque des revendications 9, 11 ou 13, **caractérisé en ce qu'**on utilise une solution d'une thromboplastine tissulaire recombinante.

**19.** Procédé selon l'une quelconque des revendications 9, 11 ou 13, **caractérisé en ce qu'**on utilise une solution d'un variant biologiquement actif d'une thromboplastine tissulaire.